(19) Europäisches Patentamt
European Patent Office
Office européen des brevets

(11) EP 3 736 303 A1

(12) **EUROPEAN PATENT APPLICATION**

(43) Date of publication:
**11.11.2020 Bulletin 2020/46**

(21) Application number: **19172816.1**

(22) Date of filing: **06.05.2019**

(51) Int Cl.:
*C08G 63/08* (2006.01) *A61K 47/34* (2017.01)
*A61L 27/12* (2006.01) *A61L 27/18* (2006.01)
*A61L 31/06* (2006.01) *C08G 63/64* (2006.01)
*C08G 63/82* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Evonik Operations GmbH**
**45128 Essen (DE)**
- **Friedrich-Schiller-Universität Jena**
**07743 Jena (DE)**

(72) Inventors:
- **WINDHAB, Norbert, Dr.**
**65719 Hofheim (DE)**
- **BINDL, Martin, Dr.**
**68239 Mannheim (DE)**
- **KARAU, Andreas, Dr.**
**63571 Gelnhausen (DE)**
- **SEIBEL, Manuel, Dr.**
**64625 Bensheim (DE)**
- **ENDRES, Thomas, Dr.**
**Hoover, AL 35226 (US)**
- **SCHUBERT, Ulrich Sigmar, Prof.**
**07743 Jena (DE)**

(74) Representative: **Evonik Patent Association**
**c/o Evonik Industries AG**
**IP Management**
**Bau 1042A/PB 15**
**Paul-Baumann-Straße 1**
**45772 Marl (DE)**

(54) **NOVEL CATALYZED SYNTHESIS OF BIODEGRADABLE POLYESTERS**

(57) The invention relates to a catalytic process for preparing polyester by ring-opening polymerization of at least one substituted glycolide of formula (I) or copolymerization of at least one substituted glycolide of formula (I) with trimethyl carbonate and/or at least one lactone of formula (II), wherein a strontium(II) alkoxide $Sr(OR^3)_2$, is used as a catalyst.

EP 3 736 303 A1

## Description

### Field of the invention

The present invention refers to a novel catalytic process for preparing biodegradable polyesters using strontium alkoxide as a catalyst, polyester polymers obtainable by this process and the use thereof in medical devices and controlled release systems.

### Background of the invention

Polyesters such as polylactide (PLA) and polyglycolide (PGA) polymers belong to the most widely established biodegradable materials. Due to their good biocompatibility, safety and attractive mechanical and processual properties, PLA- and PGA-based polymers and composites comprising such polymers are used in various applications, particularly in biomedical and pharmaceutical areas. Thus, polylactide and polyglycolide polymers have been employed in production of bio-resorbable sutures, bioabsorbable implants for bone fixation, scaffolds for tissue engineering and carriers for controlled drug release systems.

### Description of the prior art

The most common approach to synthesize PLA and PGA polymers is ring-opening polymerization (ROP) of lactide or glycolide catalysed by tin octanoate (also known as tin(II) 2-ethylhexanoate). This highly active catalyst can provide PLA polymers with high molar mass in bulk or in solution. However, increasing concerns about catalyst residuals in polymers and safety have led to development of other catalytic process types recently. Thus, metal alkoxides such as aluminium, lanthanum, yttrium or calcium alkoxides have been reported to catalyse ROP reaction of lactide and glycolide. Each individual type of catalyst may provide polymers with different molecular mass distribution, stereochemistry or other product features important for targeted application of the corresponding polymer product. Therefore, the search for novel catalysts for ROP of lactide and glycolide and for novel polyester properties remains an important goal of research and development process.

Strontium catalysts are quite rarely used for ROP reactions.

CN 105566615 A discloses ring-opening polymerization of glycolide under harsh conditions using $Sr(OAc)_2$ (embodiments 13, 14), SrO (embodiment 15) or $SrCl_2$ (embodiments 17,18), respectively, as a catalyst at 170-210 °C. The polydispersity index ($M_w/M_n$) of the resulting PGA polymers ranges from 1.50 to 2.18 with number-average molecular masses from 16.752 to 51.075 g/mol.

CN 1831027 A discloses preparation of polylactic acid by ROP of lactide catalyzed with bimetallic aluminium/strontium complexes. Polymerization is carried out at very high temperatures (165-190 °C) and provides polymers with molecular masses of 120.000-600.000 g/mol.

Z. Tang et al. disclose in Table 2, examples 12-14 of J. of Polym. Sci. Part A: Polym. Chem., 2003, 41, 1934-1941 ring-opening polymerization of ε-caprolactone using strontium isopropoxide as a catalyst. The reactions in these examples were carried out at 40 °C for 6 hours in toluene as a solvent. The authors demonstrate, that the polymerization of caprolactone was effective but resulted in much broader molecular weight distributions ($M_w/M_n$ = 3.37-7.37) than with the other catalyst employed, amino isoproxyl strontium (Sr-PO). This latter catalyst is shown to be an effective catalyst for ring-opening polymerization of lactide to polyesters with narrow molecular weight distributions. Therefore, Tang et al. teach away from using strontium isopropoxide for polymerization of lactide in order to obtain polymers with a narrow molecular weight distribution. Nor do they disclose using any alcohols as co-catalysts in addition to strontium catalyst.

The catalysts used for ROP of lactide or glycolide usually remain in the resulting polymer and may influence the final application it is used for, e.g. facilitate the process of bio-degradation of such polymers. Additionally, the potential toxicity of such catalysts remaining in the polymer may be very critical for the use of such polymers in medical applications.

Strontium (Sr) is an alkaline earth metal with atomic number 38. The similarity of Sr with calcium (Ca) enables Sr to be efficiently incorporated into the mineral phase of bone. The positive effect of Sr on bone homeostasis was revealed by studies carried out in the 1950's [Shorr E, Carter AC. Bull Hosp Jt Dis 1952, 13, 59-66.]. Since 2004, Strontium Ranelate (SrRan) has been approved for treatment of osteoporosis in several European countries. Studies on healthy animals have shown that oral administration of SrRan in relation to insertion of bone integrating implants improved the peri-implant bone volume and enhanced implant pull-out strength [Maïmoun L et al. Bone, 2010, 46, 1436-1441 ; Li Y et. al. Clin Oral Implants Res 2012, 23, 1038-1044]. A growing body of research shows that low doses of strontium may lead to increased bone formation and decreased bone resorption, may be clinically effective in preventing bone loss and formation of fractures and increases bone mineral density (BMD), which in turn is an efficient means for preventing osteoporosis [Clinical Nutrition 2007, vol. 26, pp. 193-207].

Therefore, using strontium compound as a catalyst for preparing biodegradable polymers not only would not

have a negative effect associated with any toxicity, but may have an additional benefit assignable to the positive effect on bones health due to strontium remaining in the polymer.

**Problem and Solution**

There is a need for a novel process for ring-opening polymerization of lactide or glycolide and co-polymerization of these compounds with other suitable monomers under mild reaction conditions to provide biodegradable polymers with a desired, controlled by process, e.g. low polydispersity index ($M_w/M_n$) and relatively low molecular weight, which do not contain any toxic or potentially dangerous metal or mineral residues. There is also a need for biodegradable polyesters with novel and unique properties, which can be applied in novel biomedical and pharmaceutical applications such those of amorphic and osteoconductive bio-functionally doped materials for better healing and growth morphological results.

The problem was solved by a
catalytic process for preparing polyester by ring-opening polymerization of at least one substituted glycolide of formula (I)

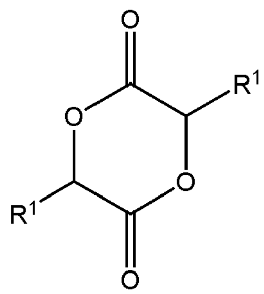

(I) ,

wherein $R^1$ is H or a hydrocarbon radical with 1-10 carbon atoms,
or copolymerization of at least one substituted glycolide of formula (I) with trimethylene carbonate and/or at least one lactone of formula (II),

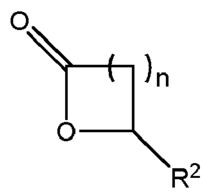

(II) ,

wherein n = 1-10, $R^2$ is H or a hydrocarbon radical with 1-10 carbon atoms,
and a strontium(II) alkoxide $Sr(OR^3)_2$, wherein
$R^3$ is a hydrocarbon radical with 1-30 carbon atoms,
is used as a catalyst.

The term "hydrocarbon radical" in the present patent application refers to alkyl-, alkenyl-, alkynyl-, aryl- radicals and corresponding mixed forms thereof, e.g. radicals containing both aryl and alkyl groups. Such hydrocarbon radicals may be linear or branched and can also comprise heteroatoms other than carbon (C) and hydrogen (H), such as nitrogen (N), oxygen (O), sulphur (S), and phosphorus (P).

**Monomers**

In the substituted glycolide of formula (I) $R^1$ is preferably H or an alkyl group with 1-6 carbon atoms such as methyl, ethyl, n-propyl, iso-propyl, n-butyl, iso-butyl, tert-butyl, pentyl, n-hexyl or cyclohexyl. If $R^1 \neq$ H, all possible stereoisomers of compound of formula (I) can be employed, such as (L, L), (D,D), (D,L) or (L,D)-isomers or mixtures thereof. Most preferably, $R^1$ = H or $CH_3$ and the substituted glycolide of formula (I) is glycolide (I, $R^1$ = H) or lactide (I, $R^1 = CH_3$).

In the lactone of formula (II), n is preferably 1-5 and $R^1$ is preferably H or an alkyl group with 1-6 carbon atoms such as methyl, ethyl, n-propyl, iso-propyl, n-butyl, iso-butyl, tert-butyl, pentyl, n-hexyl or cyclohexyl. If $R^2 \neq H$, all possible stereoisomers of lactone of formula (II) can be employed, such as (L)-, (D)-isomers or a mixture thereof. Most preferably, the lactone of formula (II) is chosen from the group consisting of β-propiotactone (n = 1, $R^2$ = H), γ-butyrolactone (n = 2, $R^2$ = H), δ-valerolactone (n = 3, $R^2$ = H), ε-caprolactone (n = 4, $R^2$ = H), δ-caprolactone (n = 3, $R^2$ = Me), δ-decalactone (n = 3, $R^2$ = n-$C_5H_{11}$) and a mixture thereof.

Additionally to compounds of formula (I) and optionally compounds of formula (II), cyclic carbonates such as trimethylene carbonate (also known as 1,3-propylene carbonate and 1,3-dioxan-2-on) can be used as a monomer in the process according to the invention.

**Catalyst**

The hydrocarbon radical $R^3$ in strontium(II) alkoxide $Sr(OR^3)_2$ used in the process of the present invention is preferably chosen from the alkyl group consisting of methyl, ethyl, n-propyl, iso-propyl, n-butyl, iso-butyl, tert-butyl, n-pentyl, n-hexyl, cyclo-hexyl, 2-ethylhexyl, n-octyl, n-decyl. Most preferably, strontium(II) isopropoxide ($R^3$ = iso-propyl) is employed as a catalyst in the process of the invention.

Strontium(II) alkoxide can be synthesized by any suitable procedure, such as by a reaction of metallic strontium with the corresponding alcohol. Another possible synthesis method is a full or partial alkoxy-ligand substitution in a reaction of strontium(II) alkoxide derived from one alcohol with another alcohol. Thus, a reaction of strontium(II) methylate with ethanol would lead to a partial or complete substitution of methoxy-ligands by ethoxy-groups.

Strontium(II) alkoxide is a highly reactive compound, e.g. prone to hydrolysis. Therefore, particular care should be taken to carry out the polymerization process of the present invention in the absence of water in order to prevent the catalyst from deactivation.

Strontium(II) alkoxide is employed in catalytic quantities in ROP reaction according to the invention. The molar ratio of the sum of all employed glycolides of formula (I), trimethylene carbonate and lactones of formula (II) to the strontium(II) alkoxide is preferably from 10.000 : 1 to 5 :1, more preferably from 1.000 : 1 to 10 : 1, most preferably 500 : 1 to 20 : 1 (mol : mol).

**Alcohol**

Surprisingly, it was found that the addition of relatively small amounts of alcohols to the reaction mixture comprising the compound of formula (I) and optionally compounds of formula (II) and/or trimethylene carbonate along with strontium(II) alkoxide leads to a significant change in the polymerization reaction outcome, when compared with a similar reaction carried out without any alcohols. Thus, a lower molar mass and lower polydispersity index of the resulting polymers may be achieved with the use of such alcohols.

Thus, preferably an alcohol $R^4OH$, wherein $R^4$ is a hydrocarbon radical with 1-30 carbon atoms, more preferably with 3-20 carbon atoms, is present in the reaction mixture comprising glycolides of formula (I), strontium(II)alkoxide $Sr(OR^3)_2$, optionally trimethylene carbonate and/or lactones of formula (II) and/or a solvent at the start of the polymerization process. This alcohol, in addition to the Sr-coordinated alcoholate, defines alpha end groups of the respectively initiated polymer chain.

Preferably, the following alcohols can be employed in the inventive process: 1-propanol, 2-propanol, 1-butanol, 2-butanol, 1-pentanol, 1-hexanol, 1-heptanol, 1-octanol, 1-nonanol, 1-decanol, 1-undecanol, 1-dodecanol, 1-tridecanol, 1-tetradecanol, 1-pentadecanol, 1-hexadecanol. Most preferably, 1-dodecanol is used.

The molar ratio of alcohol $R^4OH$ to the strontium(II)alkoxide $Sr(OR^3)_2$ employed can be from 0.1:1 to 10 :1 (mol : mol).

**Polymerization process**

The process according to the invention can be carried out in bulk, without using any solvent or in an appropriate solvent. In the latter case, the solvent is preferably an aprotic solvent, which can be chosen from the group consisting of halogenated or non-halogenated aliphatic or aromatic hydrocarbons, ethers, esters, ketones, amides, nitriles, sulfoxides, nitro-compounds and mixtures thereof. For example, the solvent used can be pentane, cyclopentane, hexane, cyclohexane, heptane, octane, decane, dodecane, chloroform, dichloromethane, benzene, toluene, xylene, diethyl ether, methyl tert-butyl ether, tetrahydrofuran, 1,4-dioxane, ethyl acetate, acetone, dimethylformamide, acetonitrile, dimethyl sulfoxide, nitromethane, propylene carbonate. Most preferably, the reaction is carried out without any solvent or in the presence of toluene as a solvent.

If a solvent is used in the process of the invention, the concentration of the reactants in the reaction mixture prior to start of the polymerization process can be varied in a wide range. Preferably, the sum of molar concentrations

of all employed glycolides of formula (I), trimethylene carbonate and lactones of formula (II) in the solution prior to start of the polymerization process is from 0.1 to 10 mol/L, more preferably from 0.2 to 5 mol/L.

The reaction temperature adjusted during the process of the invention depends on the desired polymer distribution and melting point of the used monomers and can usually range from 0 °C to 250 °C and is preferably from 15 to 220 °C, more preferably from 20 °C to 200 °C.

The process according to the invention can be carried out under normal pressure (1 atm), under reduced pressure of less than 1 atm or under elevated pressure of more than 1 atm. Most preferably, the process is conducted under ambient pressure of approximately 1 atm. In this case, the reaction temperature preferably does not exceed the boiling point at ambient pressure of any reactant or solvent used in the process.

The process of the present invention can be conducted continuously, semi-continuously or discontinuously (batch wise) as the underlying chemical reaction is very close to ideal living polymerization with no cyclic reaction products present or trans-esterification occurring. The reaction time (for a discontinuous process) or the mean residence time in reactor (for a continuous or semi-continuous process) can vary from 0.1 minutes to 5 hours, preferably from 1 minute to 120 minutes.

**Polyester**

The invention provides polyester with a Sr content of 10 ppm to 1 % by weight, comprising ester units of formula (III) and optionally ester units of formula (IV) and/or of formula (V):

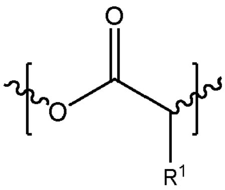
(III)

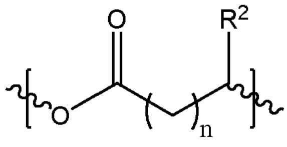
(IV)

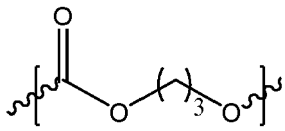
(V)

wherein the polymer species present most abundantly in the polyester, as determined by MALDI TOF mass spectrometry method, has a polymer end group of formula (VI), (VII) or (VIII):

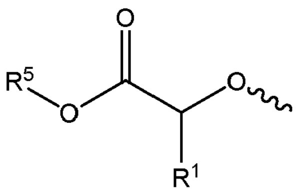
(VI)

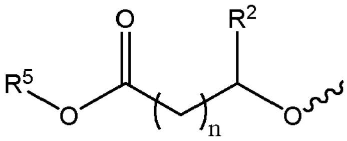
(VII)

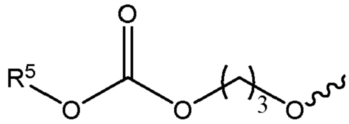
(VIII)

wherein $R^1$ and $R^2$ are independent from each other, H or a hydrocarbon radical with 1-10 carbon atoms; $R^5$ is a hydrocarbon radical with 1-30 carbon atoms, n = 1-10.

Ester units of formula (III) originate from polymerization of substituted glycolide of formula (I); ester units of formula (IV) are formed by polymerization of lactones of formula (II), ester units of formula (V) are formed by polymerization of trimethylene carbonate.

Radical $R^5$ in polymer end groups of formula (VI), (VII) and (VIII) may originate from strontium alkoxide catalyst (in this case, $R^5 = R^3$) and/or from the alcohol (in this case, $R^5 = R^4$) employed.

The polymer species present most abundantly in the polyester can be determined by analyzing the mass spectrum of the polymer obtained by Matrix-Assisted Laser-Desorption-Ionisation with Time of Flight Analysis (MALDI TOF) mass spectrometry (MS). The peak with the highest intensity in the mass spectrum is considered to correspond to the species present most abundantly in the analyzed polyester. Identification of the chemical structure of the polyester end groups can be achieved for example by analyzing the isotopic patterns in mass spectrum. Thus, similarity of the experimental mass spectrum peak distribution with the one calculated for the molecule of particular chemical structure can serve as a proof for this particular chemical structure present in the polymer mass (see Figure 1D for an example).

The polyester according to the present invention is obtainable for example by the process of the present invention, described in detail above.

Thus, due to the use of strontium(II) alkoxide $Sr(OR^3)_2$ as a catalyst and optionally additionally $R^4OH$ in the

process of the present invention, the polyester obtainable by a process according to the present invention may show polymer end groups of formula (VIa, $R^5 = R^3$) or (VIb, $R^5 = R^4$), respectively, originating from the substituted glycolide of formula (I), polymer end groups of formula (VIIa, $R^5 = R^3$) and (VIIb, $R^5 = R^4$), respectively, originating from the substituted lactone of formula (II) or polymer end groups of formula (VIIIa, $R^5 = R^3$) and (VIIIb, $R^5 = R^4$), respectively, originating from trimethylene carbonate:

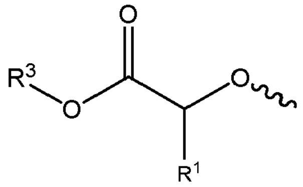

(VIa)

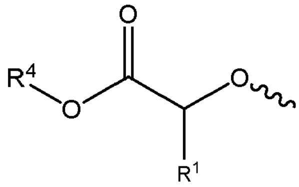

(VIb)

(VI): Polyester end group originating from substituted glycolide of formula (I)

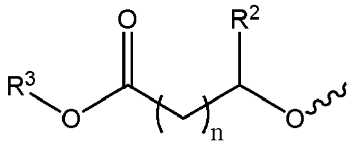

(VIIa)

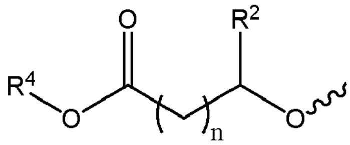

(VIIa)

(VII): Polyester end group originating from lactone of formula (II)

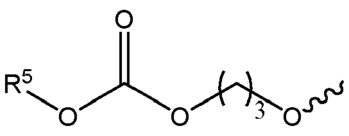

(VIIIa)

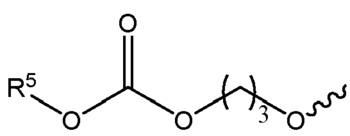

(VIIIb)

(VIII): Polycarbonate end group originating from of trimethylene carbonate.

Use of other than alkoxides strontium compounds as catalysts for ring-opening polymerization of compounds of formula (I), (II) or trimethylene carbonate, would lead to different than those of formula (VI), (VII) or (VIII) polyester end groups. Thus, described in CN 105566615 A $Sr(OAc)_2$ in ROP of lactide would lead to polymers of formula (IX) with hydroxyl and acetoxy end-groups:

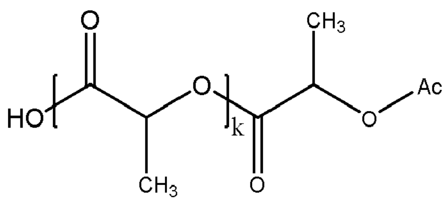

(IX)

The polyester of the present invention has a Sr content of 10 ppm to 1 % by weight, preferably of 100 ppm to 0.8 % by weight, more preferably of 200 ppm to 0.7 % by weight, still more preferably of 500 ppm to 0.5 % by weight. The abbreviation "ppm" refers to parts per million. The Sr content can be measured by atom adsorption spectroscopy (AAS) or inductively coupled plasma optical emission spectrometry (ICP-OES) method.

The polyester according to the invention preferably has a weight average molecular weight ($M_w$) of 800 g/mol to 250.000 g/mol, more preferably of 1000 g/mol to 120.000 g/mol, even more preferably of 1500 g/mol to 60.000 g/mol. The polyester of the invention may have a number average molecular weight ($M_n$) of 500 g/mol to 200.000 g/mol, more

preferably of 800 g/mol to 100.000 g/mol, even more preferably of 1000 g/mol to 50.000 g/mol, still even more preferably of 1000 g/mol to 25.000 g/mol. Both the polymer weight average and the number average molecular weights can be measured by gel permeation chromatography (GPC), also known as size-exclusion chromatography (SEC) calibrated using polystyrene or poly(lactic acid)-standards and with tetrahydrofuran (THF) used as eluent.

The polydispersity index D is used as a measure of the broadness of a molecular weight distribution of a polymer and is defined by the following equation:

$$\text{Polydispersity index D} = M_w/M_n$$

The polydispersity index of the inventive polyester is preferably in the range 1.03-4.0, more preferably 1.05-3.0, more preferably 1.07-2.0, even more preferably 1.1-1.5.

Most preferably, the polyester of the present invention has a polydispersity index of D = 1.1 - 1.5 and a number average molecular weight of $M_n$ = 1.000-25.000 g/mol.

The polyester according to the present invention preferably shows a characteristic peak in infra red (IR) spectrum at 1749±5 $cm^{-1}$ corresponding to carbonyl groups present in the lactic acid based polymer.

The glass transition temperature $T_g$ of the polyester may be in the range of -80 °C - 80 °C, preferably -60 °C - 60 °C. Most preferably, the glass transition temperature $T_g$ of the polyester is in the range of 34 °C - 48 °C, still more preferably 35 °C - 47 °C. The glass transition temperature $T_g$, can be measured as described in European Pharmacopeia 7.0 (EP) chapter 2.2.34.

The polyester according to the present invention preferably has a specific surface area (BET method) of 2.0 $m^2$/g or less, more preferably of 1.5 $m^2$/g or less, even more preferably of 1.0 $m^2$/g or less, still more preferably of 0.01 $m^2$/g - 2 $m^2$/g or of 0.1 $m^2$/g - 1 $m^2$/g. The specific surface area can be determined according to the United States Pharmacopeia 36 (USP) chapter <846> and European Pharmacopeia 7.0 (EP) chapter 2.9.26. The specific surface area is determined utilizing a specific surface area detection equipment (e.g. Quantachrome Nova 2000e BET). The specific surface area can be measured using the multi-point and single-point determination using the static-volumetric method (Method II). Prior to the measurement the sample is degassed at 20°C under vacuum.

The polyester of the invention preferably has an inherent viscosity IV of a 0.1 % solution in chloroform of 0.1 dL/g - 2.0 dL/g, 0.12 dL/g - 1.2 dL/g, 0.14 dL/g - 1.0 dL/g, 0.16 dL/g - 0.44 dL/g, 0.16 dL/g - 0.24 dL/g. The determination of the inherent viscosity can be performed in a Ubbelohde viscometer of type 0c at 25 ± 0.1 °C utilizing a polymer sample concentration of 0.1% in chloroform.

The invention further provides polyester with a Sr content of 10 ppm to 1 % by weight obtainable by the inventive process. Preferably, this polyester possesses some or all of the preferable polymer properties described for the polyester according to the present invention.

Preferable, but not limiting examples of such polyesters include poly(L-lactide), poly(D,L-lactide), poly(L-lactide-co-D,L-lactide), poly(D,L-lactide-co-glycolide), poly(glycolide), poly(L-lactide-co-glycolide), poly(glycolide-co-trimethylene carbonate), poly(L-lactide-co-trimethylene carbonate), poly(D,L-lactide-co-trimethylene carbonate), poly(L-lactide-co-caprolactone).

If the polyester is a poly(lactide-co-glycolide), than a molar ratio of lactide : glycolide is preferably from about 80 : 20 to about 40 : 60, most preferably with a molar ratio of lactide : glycolide from about 78 : 22 to about 45 : 55.

The polyester obtainable by the inventive process has a Sr content of 10 ppm to 1 % by weight, preferably of 100 ppm to 0.8 % by weight, more preferably of 200 ppm to 0.7 % by weight, still more preferably of 500 ppm to 0.5 % by weight.

The polyester obtainable by the inventive process preferably has a polydispersity index of D = 1.1-1.5 and a number average molecular weight of $M_n$ = 1.000 g/mol-25.000 g/mol.

**Articles comprising polyester**

The invention further provides bio-resorbable surgical article such as a filament, a rod, a stent or a prostheses, a suture, an implant for bone fixation, bone cement, a scaffold for tissue engineering, a component of controlled drug release system, an in vitro or in vivo cell culture media, a cell culture matrix, a bio-adherent mixture such as bio-glue, a dental or a facial implant, a tendon repair structure, an implant or medical device coating comprising the polyester according to the invention. The term "bio-resorbable" used for description of polymers, controlled release systems, surgical articles and suchlike, means that the polyester of the invention, comprising ester units of formula (III), is after implantation or injection in the human body or in the body of an animal in contact with the body fluids broken down into oligomers in a slow hydrolytic reaction. Hydrolysis end products such as lactic acid or glycolic acid, the $R^4OH$ and $R^3OH$ alcohols and the catalytic Sr-adducts are metabolized into carbon dioxide and water or secreted or deposited in healthy physiological mineral compartments. Other exchangeable expressions for the term "bio-resorbable" which are often

used are "resorbable", "bio-degradable" or "adsorptive".

The systems, e.g. comprising special polymers, aimed at targeted transport and delivery of pharmaceutically active compounds in order to facilitate their pharmacokinetics and achieving the desired therapeutic effect thereof in the human or animal body, are referred to as controlled drug release systems.

Articles comprising the polyester of the present invention include polymer composites, which may comprise other than polyester polymer types, such as polyamide, polyurethane, polyetheretherketone (PEEK), and other polymers.

**Use of the polyester**

The polyester according to the invention can be used for preparing a bio-resorbable pharmaceutical active ingredient containing dosage form suitable for controlled drug release systems, drug eluting implants, resorbable implants, and coated implants, e.g. in-situ sustained release systems in the human body or in an animal body.

The polyester of the present invention can also be used for preparing a bio-resorbable medical device or surgical article such a filament, a rod, a stent or a prostheses, a suture, an implant for bone fixation, bone cement, a scaffold for tissue engineering, an in vitro or in vivo cell culture media, a cell culture matrix, a bio-adherent mixture such as bio-glue, a dental or a facial implant, a tendon repair structure, an implant or medical device coating and suchlike.

The polyester of the present invention, due to their Sr content, can further be used for specific in vivo imaging using such methods as electron density x-ray absorption and nuclear spin effects.

The polyester of the present invention can also be used to bio-activate implants and surgical intermediates for healing, in implant stabilizers and for bone repair.

The invention further provides polyester according to the invention for use in preparing a bio-resorbable pharmaceutical active ingredient containing dosage form suitable for controlled drug release systems in the human body or in an animal body.

The invention further provides polyester according to the invention for use in preparing a bio-resorbable medical device or surgical article such a filament, a rod, a stent or a prostheses, a suture, an implant for bone fixation, bone cement, a scaffold for tissue engineering, an in vitro or in vivo cell culture media, a cell culture matrix, a bio-adherent mixture such as bio-glue, a dental or a facial implant, a tendon repair structure, an implant or medical device coating.

**Examples**

**Examples 1-6**

**General procedure**

A suitable reaction vessel was charged with 1 mmol (144 mg) of L-lactide and 2 mL of anhydrous toluene to form a 0.5 mol/L solution of L-lactide in toluene.

100 μL of a stock solution containing the required quantity of $Sr(OiPr)_2$ and, if necessary, dodecanol in anhydrous toluene were added in order to start the reaction. The amounts of the employed L-lactide, strontium catalyst and alcohol corresponded the molar ratio [lactide]:[ $Sr(OiPr)_2$]:[dodecanol] specified in Table 1.

The ring-opening polymerization (ROP) proceeded at room temperature (25 °C) under stirring inside the glovebox for a time, specified in Table 1. 150 μL aliquots were withdrawn regularly and quenched by addition of 50 μL of a solution of benzoic acid (4 equivalents with respect to $Sr(OiPr)_2$) in toluene. $^1$H-NMR and size-exclusion chromatography (SEC) analyses were performed from the quenched reaction mixtures in order to assess the monomer conversion, the average number molar mass, and the polydispersity index of the resulting polylactide after corresponding reaction time are specified in Table 1.

**Table 1**

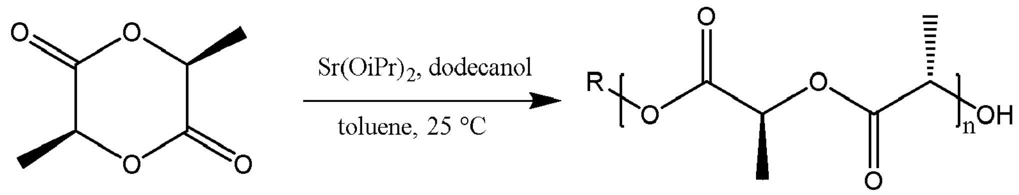


| Example | [M] : [Sr] : [dodecanol] [mol : mol : mol] | t, [min] | Conversion, [%] | $M_n$, [g/mol] | $M_w/M_n$ |
|---|---|---|---|---|---|
| 1 | 20 : 1 : 1 | 0.17 | > 99 | 1.000 | 1.41 |
| 2 | 20 : 1 : 0 | 1 | 96 | 3.200 | 1.21 |
| 3 | 100 : 1 : 1 | 15 | 99 | 13.000 | 1.16 |
| 4 | 100 : 1 : 0 | 15 | 76 | 16.000 | 1.16 |
| 5 | 200 : 1 : 1 | 90 | 72 | 20.000 | 1.29 |
| 6 | 200 : 1 : 0 | 90 | 52 | 19.000 | 1.27 |

As it can be seen from Table 1, $Sr(OiPr)_2$ can successfully be used as a catalyst in ring-opening polymerization of lactide. With sufficient catalyst used, very high conversions of lactide can be achieved after relatively short period of time at 25 °C (examples 1-3). With lower catalyst loadings, more time is needed for the completion of the reaction at this temperature (examples 4-6). Relatively low number average molecular weight of polymers (1.000-20.000 g/mol) and very narrow molecular weight distribution (low polydispersity index = 1.16-1.41) resulted in all cases (examples 1-6). Addition of catalytic quantities of dodecanol to the reaction mixture led to significant change of the reaction outcomes. Thus, lactide conversion increased significantly with this alcohol added (example 3 vs. example 4 and example 5 vs. example 6). In examples 1 and 3, substantially lower number average molecular weights have resulted from the reaction as in comparable examples 2 and 4 without using an alcohol.

**Example 7**

*Synthesis of PLA with high strontium content*

Corresponding to an initial molar ratio of [L-lactide] : [$Sr(OiPr)_2$] of 20 : 1, 720 mg (5 mmol) of L-lactide were dissolved in 9.54 mL of anhydrous toluene, and 460 μL of a stock solution containing 105 mg (0.51 mmol) $Sr(OiPr)_2$ in 1 mL of anhydrous toluene were added. After 60 seconds, the polymerization mixture was quenched with a solution of benzoic acid (4 equivalents with respect to $Sr(OiPr)_2$) in anhydrous toluene, and a sample for the determination of the monomer conversion (by $^1$H-NMR) was taken. The crude PLA was precipitated into cold diethyl ether (-22°C), centrifuged at 5000 rpm for 5 minutes and the supernatant was removed. Afterwards the purified polyester was dried at 40 °C in a vacuum oven overnight. Conversion of L-lactide was 96%. Isolated Yield was 88 %.
$^1$H NMR (300 MHz, $CDCl_3$, δ): 1.51 (d, $-CH(CH_3)_2$), 1.60 (d, $-OCHCH_3-$), 5.05 (m, $-\underline{CH}(CH_3)_2$), 5.18 (q, $-O\underline{CH}CH_3-$). SEC (THF, PLA calibration): $M_n$ = 1.7 kg $mol^{-1}$; $M_w/M_n$ = 1.17. MALDI TOF MS (DCTB, NaI): $M_n$= 2.4 kg $mol^{-1}$; $M_w/M_n$ = 1.16.

Some characteristic properties of the resulting polymer were observed by analyzing mass spectra and IR-spectra of this polymer.

**Figure 1A** shows a SEC elugram of the polymer prepared in example 7 (A);
**Figure 1B** shows a MALDI TOF mass spectrum (DCTB, NaI) of the same polymer;
**Figure 1C** shows a zoom into the most abundant region of the mass spectrum of polymer from example 7;
**Figure 1D** shows an overlay of the experimental and calculated isotopic patterns for the most abundant peak and assignment to the schematic representation of the polymer structure.

Analysis of the MALDI TOF mass spectrum of the polymer prepared in example 7 shows characteristic molar masses of polymer species found in the resulting polymer mix. The difference in $\Delta m/z$ of 144 between two adjacent peaks in the mass spectrum corresponds exactly to the mass of a lactide monomer, whereas $\Delta m/z$ of 72 corresponds to the single lactic acid unit (Figure 1C). Therefore, the polymer species from example 7 consist of "dimer" as well as single lactic acid units. Apparently, this is characteristic for the mechanism of strontium catalyst's action occuring in the ROP reaction.

Another interesting aspect of the polymer species revealed by observing mass spectra of the resulting polymers, is that the polymers show end isopropyl groups originating from the initial catalyst $Sr(OiPr)_2$ (Figure 1D).

**Figure 2** shows an ATR-IR spectrum of the PLA polymer prepared in example 7 employing a [LA]:[$Sr(OiPr)_2$] = 20:1 as well as the peak assignment. The peak at 1749 $cm^{-1}$ corresponds to carbonyl groups present in the polymer of the present invention. This peak is slightly shifted compared to the one typically found in polymers prepared using tin (II) octoate, a well-established catalyst for ROP of lactide. This specific IR peak shift results from the specific coordination of carbonyl groups with Sr in the polymer prepared according to the present invention.

**ROP with lactones**

In additional examples, $Sr(OiPr)_2$ was shown to be an efficient catalyst for polymerization of lactones and co-polymerization of lactones with substituted glycolides. Lactones, such as ε-caprotactone and δ-valerolactone, were shown to be very reactive in such reactions and lead to a broader molecular weight distribution of the resulting polymers than in the case of homopolymerization of substituted glycolides. Thus, addition of substituted lactones of formula (II) to substituted glycolides of formula (I) in ring-opening copolymerization allow additional adjusting of polydispersity index, Tg and other polymer properties.

## Claims

1. Catalytic process for preparing polyester by ring-opening polymerization of at least one substituted glycolide of formula (I)

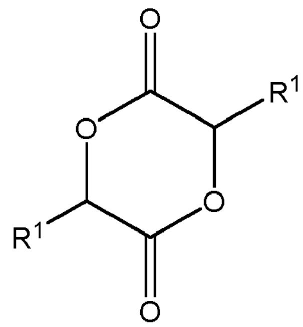

(I) ,

wherein $R^1$ is H or a hydrocarbon radical with 1-10 carbon atoms,
or copolymerization of at least one substituted glycolide of formula (I) with trimethylene carbonate and/or at least one lactone of formula (II),

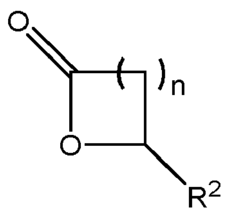

(II) ,

wherein n = 1-10, $R^2$ is H or a hydrocarbon radical with 1-10 carbon atoms,
**characterized in that**
a strontium(II) alkoxide $Sr(OR^3)_2$, wherein
$R^3$ is a hydrocarbon radical with 1-30 carbon atoms,
is used as a catalyst.

2. The process according to Claim 1,
**characterized in that** $R^1$ = H or $CH_3$ and the substituted glycolide of formula (I) is glycolide or lactide.

3. The process according to Claim 1 or 2,
**characterized in that** the lactone of formula (II) is chosen from the group consisting of β-propiolactone (n = 1, $R^2$ = H), γ-butyrolactone (n = 2, $R^2$ = H), δ-valerolactone (n = 3, $R^2$ = H), ε-caprolactone (n = 4, $R^2$ = H), δ-caprolactone (n = 3, $R^2$ = Me), δ-decalactone (n = 3, $R^2$ = n-$C_5H_{11}$) and a mixture thereof.

4. The process according to either of Claims 1 to 3,
**characterized in that** $R^3$ is chosen from the group consisting of methyl, ethyl, n-propyl, i-propyl, n-butyl, iso-butyl, tert-butyl, n-pentyl, n-hexyl, cyclo-hexyl, 2-ethylhexyl, n-octyl, n-decyl.

5. The process according to either of Claims 1 to 4,
**characterized in that**
the molar ratio of the sum of all employed glycolides of formula (I) and lactones of formula (II) to the strontium(II)alkoxide is from 1000 : 1 to 10 : 1.

6. The process according to either of Claims 1 to 5,
**characterized in that** the reaction temperature during the process is from 20 °C to 200 °C.

7. The process according to either of Claims 1 to 6,
**characterized in that** the process is carried out in an aprotic solvent chosen from the group consisting of halogenated or non-halogenated aliphatic or aromatic hydrocarbons, ethers, esters, ketones, amides, nitriles, sulfoxides, nitro-compounds and mixtures thereof.

8. The process according to Claim 7,
**characterized in that** the sum of molar concentrations of all employed glycolides of formula (I), trimethylene carbonate and/or lactones of formula (II) in the solution prior to start of the polymerization process is from 0.1 to 10 mol/L.

9. The process according to either of Claims 1 to 8,
**characterized in that** an alcohol $R^4OH$, wherein $R^4$ is a hydrocarbon radical with 1-30 carbon atoms, is present in the reaction mixture comprising glycolides of formula (I), strontium(II)alkoxide $Sr(OR^3)_2$, optionally lactones of formula (II) and/or the solvent at the start of the polymerization process.

10. The process according to either of Claims 1 to 9,
**characterized in that** the molar ratio of alcohol $R^4OH$ to the strontium(II)alkoxide $Sr(OR^3)_2$ is from 0.1 : 1 to 10 : 1.

11. Polyester with a Sr content of 10 ppm to 1 % by weight, comprising ester units of formula (III) and optionally ester units of formula (IV) and/or of formula (V):

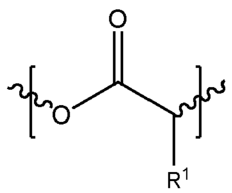
(III)

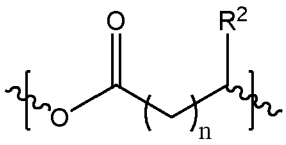
(IV)

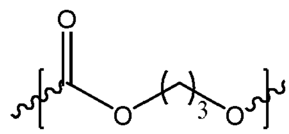
(V)

wherein the polymer species present most abundantly in the polyester, as determined by MALDI TOF method, has a polymer end group of formula (VI), (VII) or (VIII):

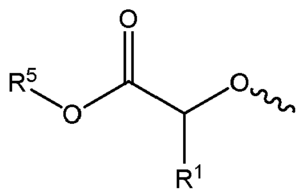
(VI)

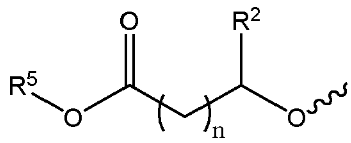
(VII)

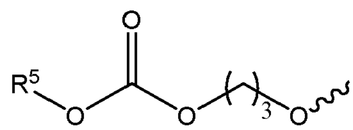
(VIII)

wherein $R^1$ and $R^2$ are independent from each other, H or a hydrocarbon radical with 1-10 carbon atoms; $R^5$ is a hydrocarbon radical with 1-30 carbon atoms, n = 1-10.

12. Polyester with a Sr content of 10 ppm to 1 % by weight,
obtainable by a process according to either of Claims 1 to 10.

13. Bio-resorbable surgical article such a filament, a rod, a stent or a prostheses, a suture, an implant for bone fixation, bone cement, a scaffold for tissue engineering, a component of controlled drug release system, an in vitro or in vivo cell culture media, a cell culture matrix, a bio-adherent mixture such as bio-glue, a dental or a facial implant, a tendon repair structure, an implant or medical device coating comprising the polyester according to either of Claims 11 or 12.

14. Polyester according to either of Claims 11 or 12 for use in preparing a bio-resorbable pharmaceutical active ingredient containing dosage form suitable for controlled drug release systems in the human body or in an animal body.

15. Polyester according to either of Claims 11 or 12 for use in preparing a bio-resorbable medical device or surgical article such a filament, a rod, a stent or a prostheses, a suture, an implant for bone fixation, bone cement, a scaffold for tissue engineering, an in vitro or in vivo cell culture media, a cell culture matrix, a bio-adherent mixture such as

bio-glue, a dental or a facial implant, a tendon repair structure, an implant or medical device coating.

Figure 1A

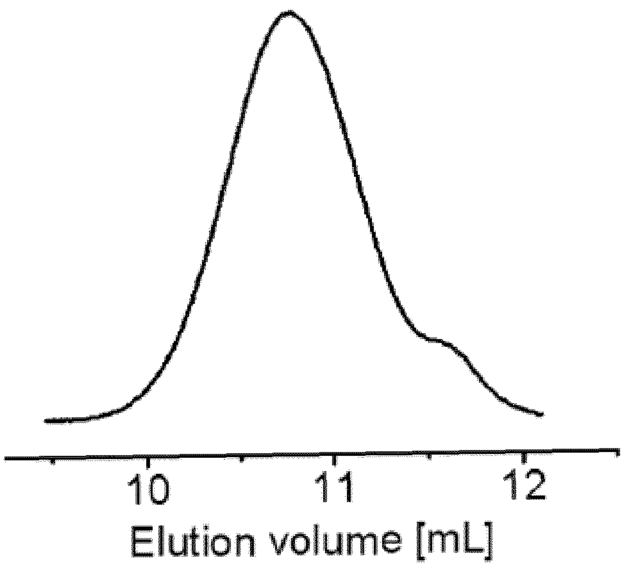

**Figure 1B**

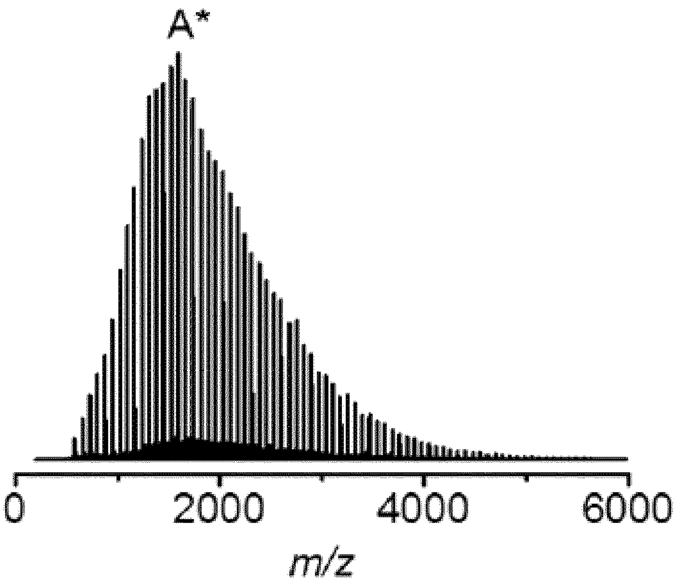

Figure 1C

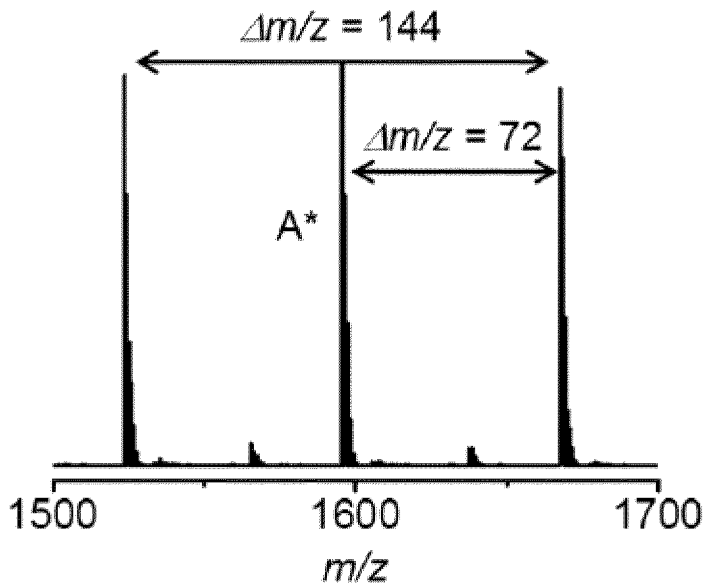

**Figure 1D**

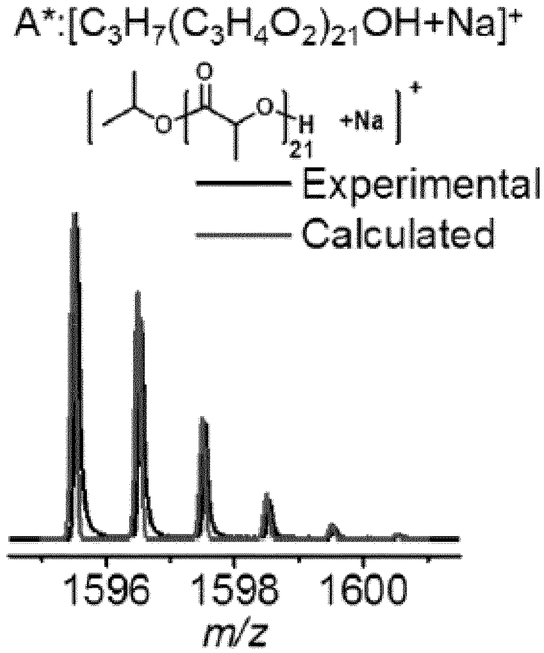
A*:[C3H7(C3H4O2)21OH+Na]+
Experimental
Calculated
1596
1598
1600
m/z

Figure 2

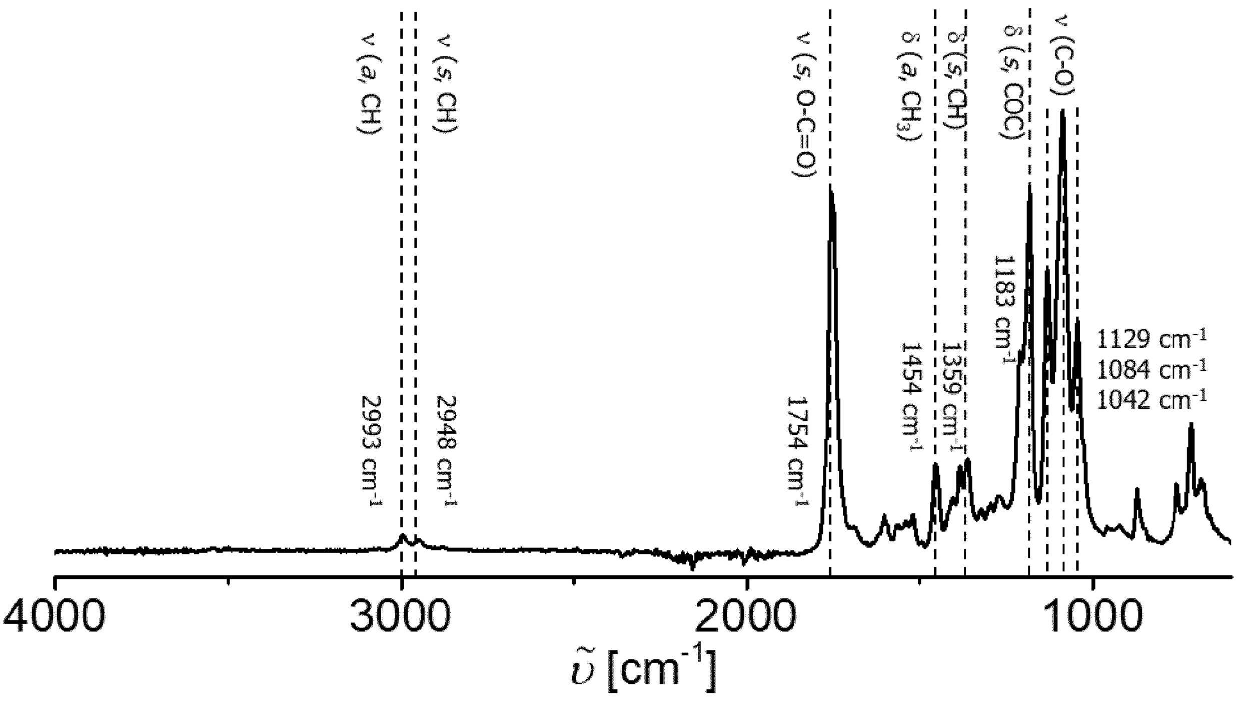

ν (a, CH)
2993 cm-1
ν (s, CH)
2948 cm-1
ν (s, O-C=O)
1754 cm-1
δ (a, CH3)
1454 cm-1
δ (s, CH)
1359 cm-1
δ (s, COC)
1183 cm-1
ν (C-O)
1129 cm-1
1084 cm-1
1042 cm-1
4000
3000
2000
1000
υ̃ [cm-1]

Europäisches Patentamt
European Patent Office
Office européen des brevets

# EUROPEAN SEARCH REPORT

Application Number
EP 19 17 2816

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br><br><br><br><br><br><br><br>Y<br>A | Zhaohui Tang ET AL: "Strontium-based initiator system for ring-opening polymerization of cyclic esters", Journal of Polymer Science Part A: Polymer Chemistry, 1 July 2003 (2003-07-01), pages 1934-1941, XP055633865, DOI: 10.1002/pola.10740 Retrieved from the Internet: URL:https://onlinelibrary.wiley.com/doi/full/10.1002/pola.10740 [retrieved on 2019-10-18] * page 1935, right-hand column, line 10 - page 1938, right-hand column, line 20; figure 4; examples 7-11; table 2 * ----- | 1-8,11, 12<br><br><br><br><br><br><br><br><br><br><br><br>13-15<br>9,10 | INV.<br>C08G63/08<br>A61K47/34<br>A61L27/12<br>A61L27/18<br>A61L31/06<br>C08G63/64<br>C08G63/82 |
| Y | EP 1 908 484 A2 (CORDIS CORP [US]) 9 April 2008 (2008-04-09) * paragraph [0118] - paragraph [0122] * ----- | 13-15 | |
| X<br><br><br>A | EP 2 253 637 A1 (TOTAL PETROCHEMICALS RES FELUY [BE]; CENTRE NAT RECH SCIENT [FR]) 24 November 2010 (2010-11-24) * paragraph [0026] - paragraph [0108]; claims 2, 10, 11; examples 21-26; table III * ----- | 11-15<br><br><br>1-10 | TECHNICAL FIELDS SEARCHED (IPC)<br>C08G<br>A61L<br>A61K |
| A | CN 107 022 070 A (UNIV NANJING TECH) 8 August 2017 (2017-08-08) * claim 5; examples 1-6; compound 16 * ----- | 1-15 | |

The present search report has been drawn up for all claims

1

| Place of search | Date of completion of the search | Examiner |
|---|---|---|
| The Hague | 24 October 2019 | Enescu, Cristina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 19 17 2816

This annex lists the patent family members relating to the patent documents cited in the above-mentioned European search report. The members are as contained in the European Patent Office EDP file on
The European Patent Office is in no way liable for these particulars which are merely given for the purpose of information.

24-10-2019

| Patent document cited in search report | Publication date | Patent family member(s) | Publication date |
|---|---|---|---|
| EP 1908484 A2 | 09-04-2008 | AU 2007216919 A1 | 24-04-2008 |
| | | CA 2605699 A1 | 06-04-2008 |
| | | CN 101156965 A | 09-04-2008 |
| | | EP 1908484 A2 | 09-04-2008 |
| | | IL 186191 A | 31-12-2013 |
| | | JP 5383993 B2 | 08-01-2014 |
| | | JP 2008110204 A | 15-05-2008 |
| | | KR 20080031835 A | 11-04-2008 |
| | | US 2008086199 A1 | 10-04-2008 |
| | | US 2013144376 A1 | 06-06-2013 |
| EP 2253637 A1 | 24-11-2010 | CN 102459284 A | 16-05-2012 |
| | | EP 2253637 A1 | 24-11-2010 |
| | | EP 2424871 A1 | 07-03-2012 |
| | | JP 6148006 B2 | 14-06-2017 |
| | | JP 2012525357 A | 22-10-2012 |
| | | KR 20120008039 A | 25-01-2012 |
| | | US 2012101233 A1 | 26-04-2012 |
| | | US 2015148516 A1 | 28-05-2015 |
| | | WO 2010125138 A1 | 04-11-2010 |
| CN 107022070 A | 08-08-2017 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 105566615 A
- CN 1831027 A


### Non-patent literature cited in the description


- *J. of Polym. Sci. Part A: Polym. Chem.,* 2003, vol. 41, 1934-1941
- **SHORR E ; CARTER AC.** *Bull Hosp Jt Dis,* 1952, vol. 13, 59-66
- **MAÏMOUN L et al.** *Bone,* 2010, vol. 46, 1436-1441
- **LI Y.** *Clin Oral Implants Res,* 2012, vol. 23, 1038-1044
- *Clinical Nutrition,* 2007, vol. 26, 193-207